(19) Europäisches Patentamt
European Patent Office
Office européen des brevets

(11) **EP 4 678 219 A1**

(12) **EUROPÄISCHE PATENTANMELDUNG**

(43) Veröffentlichungstag:
**14.01.2026 Patentblatt 2026/03**

(21) Anmeldenummer: **24188330.5**

(22) Anmeldetag: **12.07.2024**

(51) Internationale Patentklassifikation (IPC):
*A61M 60/122* (2021.01)   *A61M 60/216* (2021.01)
*A61M 60/232* (2021.01)   *A61M 60/422* (2021.01)
*A61M 60/508* (2021.01)   *A61M 60/822* (2021.01)
*F16C 32/04* (2006.01)   *H02K 7/09* (2006.01)

(52) Gemeinsame Patentklassifikation (CPC):
**A61M 60/122; A61M 60/216; A61M 60/232;
A61M 60/422; A61M 60/508; A61M 60/822;
F04D 13/0666; F16C 32/0444; F16C 32/0459;
H02K 7/09;** A61M 2205/3334; F16C 2316/18

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**GE KH MA MD TN**

(71) Anmelder: **Berlin Heart GmbH
12247 Berlin (DE)**

(72) Erfinder: **PETERS, Oliver
12247 Berlin (DE)**

(74) Vertreter: **Pfenning, Meinig & Partner mbB
Patent- und Rechtsanwälte
Joachimsthaler Straße 10-12
10719 Berlin (DE)**

(54) **STEUEREINHEIT, IMPLANTIERBARE BLUTPUMPE, PUMPENSYSTEM UND VERFAHREN**

(57)  Die Anmeldung betrifft eine Steuereinheit (100) für eine Blutpumpe (200), eine implantierbare Blutpumpe (200), ein Pumpensystem und ein Verfahren zum Ansteuern einer Blutpumpe (200), wobei die Blutpumpe (200) einen Axialflussmotor (210) mit einem Stator (220) sowie einem magnetisch lagerbaren und zum Fördern von Blut um eine Rotationsachse rotierbaren Rotor (240) umfasst, wobei der Stator (220) mindestens drei, insbesondere genau drei, koplanar um die Rotationsachse herum angeordnete Spulengruppen (230) umfasst, wobei jede der Spulengruppen (230) eine Mehrzahl von benachbarten und koplanar angeordneten Statorspulen (221) umfasst. Die Steuereinheit (100) ist dazu eingerichtet, Vorgaben für einen jeweiligen elektrischen Strom durch jede der Statorspulen (221) derart zu bestimmen, dass durch den Stator (220) eine Axialkraft (501) zum aktiven magnetischen Lagern des Rotors (240) in Axialrichtung auf den Rotor (240) ausgeübt wird und durch jede der Spulengruppen (230) eine jeweilige Tangentialkraft (502) in Tangentialrichtung auf den Rotor (240) ausgeübt wird, wobei die Steuereinheit (100) zum Bestimmen voneinander unabhängiger Vorgaben für jede der von den Spulengruppen (230) erzeugbaren Tangentialkräfte derart eingerichtet ist, dass aus den Tangentialkräften eine effektive Radialkraft (503) zum Lagern des Rotors (240) in Radialrichtung resultiert.

FIG. 2B

**Beschreibung**

Die Anmeldung betrifft eine Steuereinheit für eine Blutpumpe, eine implantierbare Blutpumpe, ein Pumpensystem, umfassend eine Blutpumpe, und ein Verfahren zum Ansteuern einer Blutpumpe.

Aus dem Stand der Technik sind verschiedene Lagerungskonzepte für als Blutpumpen ausgelegte Rotationsfluidpumpen, insbesondere für den Einsatz als Herzunterstützungsvorrichtung (kurz VAD für englisch "ventricular assist device"), bekannt. Beispielsweise sind vollmagnetisch bezüglich eines Stators gelagerte - d. h. in allen gelagerten Freiheitsgraden magnetisch gelagerte - Rotoren für solche Pumpen bekannt. Hierbei können in den verschiedenen Freiheitsgraden aktive und/oder passive magnetische Lagerungen zum Einsatz kommen. Bei der Konstruktion und im Betrieb solcher Pumpen sind verschiedene Anforderungen zu berücksichtigen und gegeneinander abzuwägen sowie Nachteile zu vermeiden oder verringern. Eine Blutpumpe soll etwa möglichst kompakt, leicht, robust sowie im Betrieb sicher, effizient und zuverlässig sein.

Für eine vollmagnetische Lagerung sind beispielsweise mehrere getrennte Magnetbaugruppen (Spulen und/oder Permanentmagnete) sowohl im Stator als auch im Rotor zur Lagerung in jeweiligen Freiheitsgraden vorgesehen worden. Bei aktiver magnetischer Lagerung (d. h. Lagerung mittels aktiv ansteuerbarer Magnetfelder) in bestimmten Freiheitsgraden können ferner Sensoren zur Erfassung von Auslenkungen und/oder Verkippungen des Rotors gegenüber einer Soll-Lage erforderlich sein. Beide Aspekte (Magnetbaugruppen und Sensorik) können die Größe und/oder Masse der Pumpe erhöhen und/oder die Effizienz im Betrieb verringern. Auch können insbesondere bei passiv magnetischer Lagerung, also mittels reiner Permanentmagnetanordnungen - Instabilitäten oder Schwingungsverhalten hinsichtlich translatorischer und/oder rotatorischer Freiheitsgrade - auftreten. Etwa tritt bei einer Auslenkung des Rotors in einer passiv gelagerten Richtung eine auslenkungsabhängige Rückstellkraft auf. Durch die passive Lagerung wird der Rotor jedoch nicht gedämpft, so dass bei entsprechender Anregung (etwa durch Strömungskräfte und/oder Patientenbewegung) ein Aufschwingen auftreten kann. Zudem hat eine hohe (maximale) Rückstellkraft in einer passiv gelagerten Richtung eine hohe Steifigkeit in einer dazu senkrechten Richtung zur Folge; eine aktive Lagerung in der letztgenannten Richtung stellt dann hohe Anforderungen an Sensorik und Regelung, was sich wie erwähnt auf Größe, Masse und/oder Effizienz der Pumpe auswirken kann. Die Auswirkung eines in einem Freiheitsgrad starken passiven Magnetlagers auf die weiteren Freiheitsgrade ist eine Folge des Earnshaw-Theorems, welches besagt, dass eine dreidimensionale Ansammlung von mehreren Dipolen, wie Punktladungen oder Permanentmagneten, einen Dipol nicht in allen Freiheitsgraden stabil lagern kann. Insbesondere gilt, dass die Summe der Steifigkeiten in Newton Dipolkraft pro Meter Auslenkung stets größer Null ist. Lagersteifigkeiten größer Null sind instabil. Das heißt, dass die instabilen Lager in einer reinen Dipolanordnung die stabilen Lager überwiegen.

Dementsprechend liegt der Anmeldung die Aufgabe zugrunde, Lösungen hinsichtlich der Konstruktion und Ansteuerung von Blutpumpen mit magnetisch lagerbarem Rotor bereitzustellen, die die genannten Anforderungen wenigstens zum Teil erfüllen und/oder die genannten Nachteile wenigstens zum Teil vermeiden oder verringern.

Zur Lösung der Aufgabe werden die Gegenstände der unabhängigen Ansprüche vorgeschlagen. Bevorzugte Weiterbildungen und optionale Merkmale ergeben sich mit den Merkmalen der abhängigen Ansprüche.

Die vorgeschlagene Steuereinheit ist zur Verwendung mit einer Blutpumpe vorgesehen, wobei die Blutpumpe einen Axialflussmotor mit einem Stator sowie einem magnetisch lagerbaren und zum Fördern von Blut um eine Rotationsachse rotierbaren Rotor umfasst, wobei der Stator mindestens drei die Rotationsachse herum angeordnete Spulengruppen umfasst, wobei jede der Spulengruppen eine Mehrzahl von benachbarten Statorspulen umfasst. Die Spulengruppen werden im Folgenden auch als Sektormotoren bezeichnet.

Die um die Rotationsachse herum angeordneten Spulengruppen und/oder die benachbarten Statorspulen der Spulengruppen können koplanar angeordnet sein. Die um die Rotationsachse herum angeordneten Spulengruppen und/oder die benachbarten Statorspulen der Spulengruppen können so angeordnet sein, dass sie eine gemeinsame Ebene schneiden und gegenüber dieser abgewinkelt sind. Die um die Rotationsachse herum angeordneten Spulengruppen und/oder die benachbarten Statorspulen der Spulengruppen können zueinander so angeordnet sein, dass sie annähernd die Form eines Kegelsegments bilden.

Der Stator kann an einem Gehäuse der Blutpumpe angeordnet sein und/oder dieses bilden. Das Gehäuse umfasst vorzugsweise einen Fluideinlass und einen Fluidauslass, die mit jeweiligen Blutgefäßen und/oder einem Herzen fluidisch verbindbar sind.

Die Rotationsachse kann sich insbesondere auf eine Hauptachse der durch die Statorspulen verursachten Rotation des Rotors beziehen und somit keine physikalische Achse der Blutpumpe sein. Die Rotationsachse definiert (in einer Soll-Position des Rotors) eine Axialrichtung sowie senkrecht dazu stehende Radial- und Tangentialrichtungen, charakterisiert durch Radial- und Tangentialvektoren, wobei die Radialvektoren durch die Rotationsachse verlaufen und die Tangentialvektoren einen Abstand von der Rotationsachse aufweisen. Die Tangentialvektoren verlaufen durch den magnetischen Schwerpunkt jedes Sektormotors.

Die Steuereinheit ist dazu eingerichtet, Vorgaben für einen jeweiligen elektrischen Strom durch jede der Statorspulen derart zu bestimmen, dass durch den Stator eine Axialkraft (im Folgenden auch als Liftkraft bezeichnet) zum aktiven magnetischen Lagern des Rotors in Axialrichtung auf den Rotor ausgeübt wird und dass durch jede der Spulengruppen eine jeweilige Tangentialkraft in Tangentialrichtung auf den Rotor ausgeübt wird.

Die Steuereinheit kann insbesondere zum Regeln einer Axialposition (Axialregelung) des Rotors zum berührungsfreien magnetischen Lagern in Bezug auf den Stator eingerichtet sein. Die Steuereinheit kann ferner zum Regeln einer Verkippung des Rotors um eine oder mehrere zur Rotationsachse im Wesentlichen senkrechte Achsen (Kippregelung) eingerichtet sein. Hierzu kann es insbesondere vorgesehen sein, dass jeder Sektormotor eine jeweilige Liftkraft (Axialkraft) derart erzeugt, dass sich ein resultierendes Drehmoment bezüglich der einen oder mehreren zur Rotationsachse im Wesentlichen senkrechten Achsen ergibt, das zum Regeln der Verkippung steuerbar ist.

Die Steuereinheit ist zum Bestimmen voneinander unabhängiger Vorgaben für jede der von den Spulengruppen erzeugbaren Tangentialkräfte derart eingerichtet, dass aus den Tangentialkräften eine effektive Radialkraft zum Lagern des Rotors in Radialrichtung resultiert. Die Steuereinheit ermöglicht damit ein Regeln einer Radialposition (Radialregelung) des Rotors und dementsprechend ein aktiv magnetisches Lagern in Radialrichtung mit den Sektormotoren des Axialflussmotors. Da hierfür keine zusätzlichen Magnetbaugruppen speziell zum Lagern des Rotors in der Radialrichtung erforderlich sind, kann die Blutpumpe entsprechend kompakt und leicht ausgeführt sein. Den oben genannten Nachteilen einer passiven Lagerung (hier in Radialrichtung) kann ferner entgegengewirkt werden. In Verbindung mit der oben genannten Kippregelung und Axialregelung kann der Rotor in allen Freiheitsgraden allein durch die Sektormotoren des Axialflussmotors aktiv magnetisch gelagert werden.

Ferner ergibt sich aus den Tangentialkräften vorzugsweise ein Drehmoment bezüglich der Rotationsachse, um den Rotor zum Fördern von Blut in Rotation zu versetzen und/oder die Rotation des Rotors zu regeln.

Am Beispiel eines Stators mit drei Spulengruppen lässt sich die Erzeugung einer effektiven Radialkraft mit Radialkraftkomponenten $F_x$, $F_y$ (senkrecht zur Rotationsachse) sowie eines Drehmoments $M_z$ bezüglich der Rotationsachse basierend auf den unabhängig vorgegebenen Tangentialkräften $F_{ti}$ (mit i = 1, 2, 3) beispielhaft folgendermaßen beschreiben:

$$F_x = F_{t1} \cdot \sin(30°) + F_{t2} \cdot \sin(150°) + F_{t3} \cdot \sin(270°) = \frac{F_{t1} + F_{t2}}{2} - F_{t3},$$

$$F_y = F_{t1} \cdot \cos(30°) + F_{t2} \cdot \cos(150°) + F_{t3} \cdot \cos(270°) = \sqrt{3}\frac{F_{t1} - F_{t2}}{2},$$

$$M_z = (F_{t1} + F_{t2} + F_{t3}) \cdot r.$$

Dabei ist r ein Radius des Kraftangriffspunkts am Rotor. Bei, beispielsweise durch Regelschleifen, gegebenen $F_x$, $F_y$ und $M_z$ setzen sich die drei $F_t$ Komponenten aus einem Drehmomentanteil $\frac{M_z}{3 \cdot r}$ und einer Projektion der kartesischen Kraftkomponente auf die tangentialen Krafteinheitsvektoren zusammen:

$$F_{tn} = \frac{M_z}{3 \cdot r} + sin(\alpha + 120° \cdot n) \cdot F_x + cos(\alpha + 120° \cdot n) \cdot F_y$$

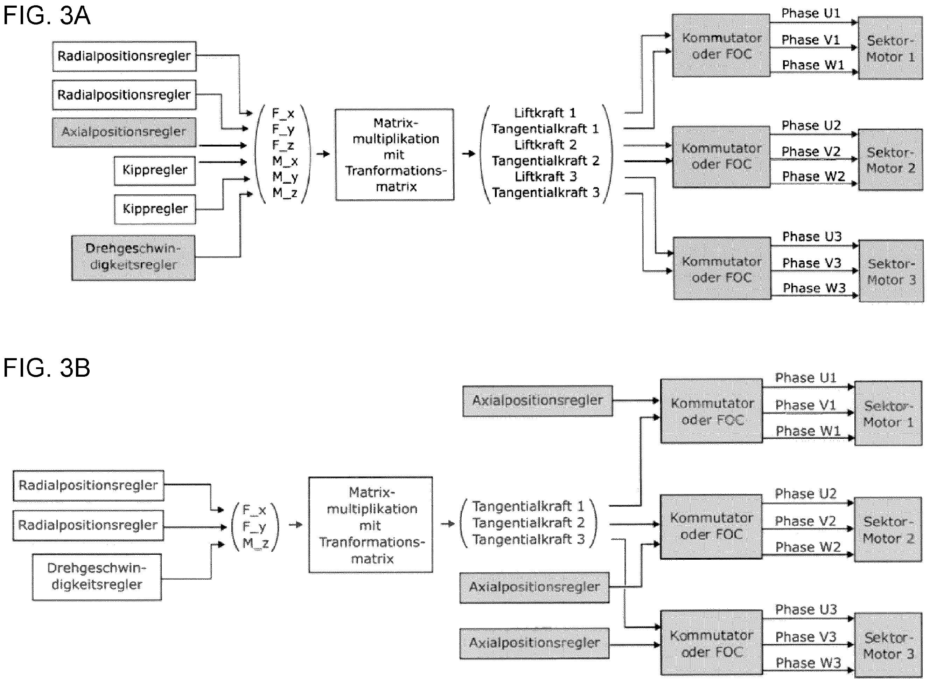

mit n= 1,2,3, und einer von der Ausrichtung des Koordinatensystems abhängigen Konstante $\alpha$. Damit entspricht die Transformationsmatrix in Fig. 3B für dieses Beispiel:

$$\begin{pmatrix} F_{t1} \\ F_{t2} \\ F_{t3} \end{pmatrix} = \begin{bmatrix} sin(\alpha + 120° \cdot 1) & cos(\alpha + 120° \cdot 1) & \frac{1}{3r} \\ sin(\alpha + 120° \cdot 2) & cos(\alpha + 120° \cdot 2) & \frac{1}{3r} \\ sin(\alpha + 120° \cdot 3) & cos(\alpha + 120° \cdot 3) & \frac{1}{3r} \end{bmatrix} \cdot \begin{pmatrix} F_x \\ F_y \\ M_z \end{pmatrix}$$

Analog hierzu setzen sich die Axialkraftkomponenten $F_{ai}$ (mit i = 1, 2, 3) ebenfalls aus einer für alle i gleichen Komponente für $F_z$ und einer koordinatensystemabhängig projizierten Komponente für $M_x$ und $M_y$ zusammen. Die Transformationsmatrix in Fig. 3A setzt sich anschließend aus den Untermatrizen zusammen und kann auch als zwei parallel durchgeführte Matrixmultiplikationen dargestellt werden.

Der Rotor umfasst typischerweise eine Permanentmagnetanordnung, insbesondere einen Magnetring, die mit einem von den Statorspulen erzeugten Magnetfeld wechselwirkt und so den Axialflussmotor bildet. Der Rotor kann ein scheibenförmiger Rotor sein, insbesondere dergestalt, dass ein Impeller des Rotors einen Durchmesser in Radialrichtung aufweist, der größer ist als eine Länge des Impellers in Axialrichtung. Bei einem scheibenförmigen Rotor dieser Art kann die beschriebene Kippregelung von besonderem Vorteil sein, da einer möglichen Instabilität in Kipprichtung entgegengewirkt wird. Der Rotor bzw. der Impeller kann auch von der axialen Länge größer sein als der Durchmesser, dann sind Kipp- und Radialregelung wegen des geringeren Hebels zwar schwächer, aber weiterhin funktionell.

Bei der Blutpumpe kann es sich insbesondere um eine implantierbare Blutpumpe (implantierbares VAD) handeln, jedoch sind auch wenigstens teilweise extrakorporale Ausführungen denkbar. Die Steuereinheit kann eine externe (im Falle der implantierbaren Blutpumpe insbesondere extrakorporale), mit der Blutpumpe mittels einer Driveline verbindbare oder verbundene Steuereinheit sein. Die Steuereinheit kann jedoch auch ganz oder teilweise in die Blutpumpe integriert und/oder mit dieser implantierbar sein. Es kann auch vorgesehen sein, dass die Steuereinheit sowohl an der Blutpumpe angeordnete als auch extern/extrakorporal angeordnete Teile umfasst.

Die Blutpumpe kann eine Zentrifugalpumpe sein, ist jedoch nicht darauf beschränkt. Da mit der aktiven Lagerung in mehreren Freiheitsgraden, insbesondere sechs Freiheitsgraden, der Abstand zwischen Schaufeln und Pumpengehäuse genau geregelt werden kann und damit klein gehalten werden kann, kann auch die Schaufelüberströmung in einer Axialpumpe klein gehalten werden, welche bei größeren Abständen zwischen Schaufel und Pumpengehäuse schneller zu ineffizienten Strömungsmaschinen führt als bei Radialpumpen. In Radialrichtung passiv magnetisch gelagerte Axialpumpen, wie beispielsweise das Herzunterstützungssystem INCOR der Berlin Heart GmbH, benötigen eine sehr steife Lagerung in Radialrichtung, um Kontakt zwischen Rotor und Pumpengehäuse zu vermeiden. Mit der aktiven Radialregelung können passive Magnetlagerkomponenten kleiner ausfallen und gleichzeitig der Schaufelspalt kleiner und konstant gehalten werden.

Die Statorspulen jeder Spulengruppe können als Phasen des jeweiligen Sektormotors eingerichtet sein. Die Steuereinheit kann zur entsprechenden Ansteuerung der Sektormotoren, etwa mittels Blockkommutierung, Sinuskommutierung und/oder Vektorregelung (field-oriented control, FOC), eingerichtet sein. Jeder Sektormotor wird hierbei von beispielsweise einer Vektorregelung angesteuert, wobei die Komponente, die in einem Rotationsmotor herkömmlicherweise Drehmomentstrom (Iq) erzeugt, im Sektormotor die Tangentialkraft erzeugt und die Komponente, welche herkömmlicherweise für Feldschwächung zum Einsatz kommt (Id), im Sektormotor lokale Axialkraft erzeugt. Analog hierzu kann jeder Sektormotor auch als lokaler Linearmotor verstanden werden. Der Stator kann insbesondere genau drei um die Rotationsachse herum angeordnete Spulengruppen umfassen. Es kann vorgesehen sein, dass jede der Spulengruppen mindestens drei benachbarte Statorspulen umfasst. Gegenüber einer Anordnung mit beispielsweise nur zwei Statorspulen je Spulengruppe kann somit etwa eine Kippregelung der beschriebenen Art besonders effektiv ermöglicht werden. Insbesondere kann jede der Spulengruppen genau drei (also insbesondere nicht mehr als drei) benachbarte Statorspulen umfassen, wodurch etwa unnötige Redundanzen und Ineffizienzen vermieden werden können. Werden nur zwei Spulen pro Spulengruppe verwendet, dann sind zwar theoretisch sechs Ströme zum Regeln von sechs Freiheitsgraden verfügbar, was das Minimum im Sinne der statischen Steuerbarkeit darstellt, allerdings sind dann die Freiheitsgrade gekoppelt, sodass beispielsweise die Kippmomente nicht in allen Rotorwinkeln unabhängig von den Radialkräften erzeugt werden können. Regelungstechnisch besitzt die Steuerbarkeitsmatrix dann einen Rang kleiner sechs.

Die Steuereinheit kann mit jeweils zwei der insbesondere drei Statorspulen jeder Spulengruppe mittels separater Anschlussleitungen elektrisch verbindbar oder verbunden sein. Mit den jeweils verbleibenden Statorspulen jeder Spulengruppe kann die Steuereinheit mittels einer gemeinsamen Anschlussleitung elektrisch verbindbar oder verbunden sein. Auf diese Weise können einzelne Leitungen der Driveline und/oder Komponenten der Leistungselektronik eingespart werden. Beispielsweise sind bei einem unabhängigen Ansteuern dreiphasiger Statorspulen in jeweils drei Sektormotoren insgesamt neun Anschlussleitungen in der Driveline und neun Halbbrücken in der Leistungselektronik erforderlich. Zum Erzeugen einer beliebigen Axialkraft und einer beliebigen Tangentialkraft mit einem Sektormotor sind jedoch nur zwei unabhängige Ströme erforderlich; der Dritte Strom ergibt sich aus diesen. Die dritte Spule jedes Sektormotors kann daher mit einer gemeinsamen Leitung verbunden und zur Leistungselektronik geführt werden, wodurch im Beispiel zwei Leitungen und zwei Halbbrücken eingespart werden können.

Entsprechend den weiter oben angestellten Überlegungen zu den regelbaren Freiheitsgraden kann die Steuereinheit zum Vorgeben des jeweiligen elektrischen Stroms durch jede der Statorspulen derart eingerichtet sein, dass der Rotor in drei rotatorischen und drei translatorischen Freiheitsgraden aktiv magnetisch gelagert ist.

Es kann alternativ vorgesehen sein, dass die Blutpumpe eine passive Magnetlageranordnung zum passiven magnetischen Lagern des Rotors in mindestens einem ersten räumlichen Freiheitsgrad umfasst. Die Steuereinheit kann dann eingerichtet sein zum Vorgeben des jeweiligen elektrischen Stroms durch jede der Statorspulen derart, dass der Rotor in den verbleibenden räumlichen Freiheitsgraden aktiv magnetisch gelagert ist.

Verschiedene Reglerkonfigurationen der Steuereinheit sind denkbar. Die Steuereinheit kann etwa eingerichtet sein zum Regeln einer Position des Rotors mittels jeweiliger Regler für jeweilige Axialpositionen des Rotors bezüglich jedes der Sektormotoren (also für jeweilige, voneinander unabhängige Liftkräfte), womit Kippwinkel des Rotors bezüglich zur Rotationsachse senkrechter Kippachsen festliegen, also nicht unabhängig geregelt werden.

Die Steuereinheit kann alternativ eingerichtet sein zum Regeln einer Position des Rotors mittels eines Reglers für eine effektive Axialposition des Rotors bezüglich des Stators insgesamt sowie mindestens eines Reglers für einen Kippwinkel des Rotors bezüglich einer zur Rotationsachse senkrechten Kippachse. Vorteilhaft ist im Vergleich zu drei Axialregelungen der Sektormotoren, dass die Regler für Verkippung und axiale Verschiebung unterschiedlich ausgelegt werden können, und somit optimal an die Masseverteilung sowie Strömungskraftverteilung des Rotors angepasst sowie voneinander entkoppelt werden können.

Die Steuereinheit kann dazu eingerichtet sein, die Vorgaben für jede der von den Spulengruppen erzeugbaren Tangentialkräfte und/oder Liftkräfte mittels einer Transformationsmatrix aus Kraft- und/oder Drehmomentvorgaben bezüglich kartesischer Koordinaten zu bestimmen.

Die Steuereinheit kann zum Regeln einer Position, insbesondere einer Translation und/oder Rotation und/oder Verkippung, des Rotors mittels einer Null-Leistungs-Regelung eingerichtet sein. Hierbei wird eine jeweilige Sollposition so eingestellt, dass auf den Rotor wirkende äußere Kräfte bzw. Momente bezüglich des zu regelnden Freiheitsgrades sich zu einer vorgegebenen Kraft, insbesondere einer Nullkraft, addieren und/oder dass eine zum Halten der Sollposition aufgewandte Leistung minimal ist (vgl. etwa das Dokument EP 3 827 852 A1).

Alternativ zu einer Regelung der im Vorausgehenden beschriebenen Art kann die Erzeugung von Radialkräften bzw. Axialkräften auch dazu verwendet werden, um lediglich bekannte radiale Strömungskräfte und/oder Kippmomente zu kompensieren. Die Steuereinheit kann dementsprechend eingerichtet sein zum Bestimmen der Vorgaben für jede der von den Spulengruppen erzeugbaren Tangentialkräfte derart, dass eine auf den Rotor wirkendende äußere Radialkraft und/oder ein auf den Rotor wirkendes äußeres Kippmoment ohne aktive Regelung kompensiert wird. Die äußere Radialkraft bzw. das äußere Kippmoment kann dabei insbesondere aus einer basierend auf einer Flussmessung und/oder Flussschätzung bestimmbaren Strömungskraft resultieren. Ebenso kann die durch die Pumpenbewegung verursachte äußere Radialkraft oder das entsprechende Kippmoment über eine Messung der Bewegung des Pumpengehäuses mittels Beschleunigungssensoren, Drehratensensoren und/oder eines Ortungssystems erfolgen.

Zum Ermöglichen einer Regelung wie oben beschrieben kann die Steuereinheit bzw. das aus Steuereinheit und Blutpumpe gebildete System ferner zum Bestimmen einer Position, insbesondere Radialposition und/oder Verkippung, des Rotors eingerichtet sein. Hierfür bestehen verschiedene Möglichkeiten, von denen einige im Folgenden erläutert werden. Die Steuereinheit kann dann zum Bestimmen der Vorgaben für jede der von den Spulengruppen erzeugbaren Tangentialkräfte und/oder Axialkräfte basierend auf der bestimmten Position eingerichtet sein.

Die Steuereinheit kann eingerichtet sein zum Bestimmen einer Position, insbesondere Radialposition und/oder Verkippung, des Rotors basierend auf einer erfassten elektromotorischen Gegenkraft (Back-EMF) und/oder basierend auf einer magnetischen Feldmessung und zum Bestimmen der Vorgaben für jede dervon den Spulengruppen erzeugbaren Tangentialkräfte und/oder Axialkräfte basierend auf der bestimmten Position. Eine Positionsbestimmung auf Grundlage der Back-EMF ist bekannt und beispielsweise in der Druckschrift EP3 827 852 A1 beschrieben. Somit wird eine Positionsmessung ohne zusätzliche Sensorik ermöglicht.

Der Stator kann einen zum Bestimmen einer Position, insbesondere Radialposition und/oder Verkippung, des Rotors eingerichteten Wirbelstromsensor umfassen. Hier kann etwa ein dedizierter Wirbelstromsensor, bevorzugt ein dreiphasiger Wirbelstromsensor, zum Einsatz kommen.

Alternativ kann ein Wirbelstromsensor durch eine oder mehrere der Statorspulen gebildet sein und dementsprechend auf zusätzliche Komponenten verzichtet werden. Eine Rotorlagemessung mit diesem Ansatz ist etwa in der Europäischen Patentanmeldung 24 153 240.7 beschrieben.

Der Rotor kann für eine solche Messung ein Wirbelstromtarget bilden oder umfassen. Das Wirbelstromtarget kann durch eine im und/oder am Rotor angeordnete elektrisch leitfähige Struktur gebildet sein. Die elektrisch leitfähige Struktur kann ein speziell zu diesem Zweck vorgesehenes Element und/oder eine (auch) zu einem anderen Zweck vorgesehene Struktur oder Komponente des Rotors sein oder umfassen. Des Weiteren kann das Wirbelstromtarget dazu eingerichtet sein, einen wirksamen Radius aufzuweisen, der sich von einem wirksamen Radius einer zum Wechselwirken mit den Statorspulen eingerichteten Rotormagnetbaugruppe unterscheidet, insbesondere größer ist. Als wirksamer oder effektiver Radius wird der Radius oder die Position bezeichnet, an welchem sich äquivalente ideale Komponenten, im Falle von Magneten sind dies Dipole, befinden würden.

Das vorgeschlagene Pumpensystem umfasst eine Blutpumpe, die einen Axialflussmotor mit einem Stator sowie einem magnetisch lagerbaren und zum Fördern von Blut um eine Rotationsachse rotierbaren Rotor umfasst, wobei der Stator mindestens drei, insbesondere genau drei, um die Rotationsachse herum angeordnete Spulengruppen umfasst, wobei jede der Spulengruppen eine Mehrzahl von benachbarten Statorspulen umfasst, und eine Steuereinheit der vorgeschlagenen Art. Es ist ersichtlich, dass die Steuereinheit in diesem Pumpensystem ihre oben diskutierten Eigenschaften und Vorteile entfaltet.

Entsprechend wird auch ein Verfahren zum Ansteuern einer Blutpumpe vorgeschlagen, wobei die Blutpumpe einen Axialflussmotor mit einem Stator sowie einem magnetisch lagerbaren und zum Fördern von Blut um eine Rotationsachse rotierbaren Rotor umfasst, wobei der Stator mindestens drei, insbesondere genau drei, um die Rotationsachse herum angeordnete Spulengruppen umfasst, wobei jede der Spulengruppen eine Mehrzahl von benachbarten Statorspulen umfasst.

Das Verfahren umfasst:

Bestimmen von Vorgaben für einen jeweiligen elektrischen Strom durch jede der Statorspulen derart, dass

durch den Stator eine Axialkraft zum aktiven magnetischen Lagern des Rotors in Axialrichtung auf den Rotor ausgeübt wird,
durch jede der Spulengruppen eine jeweilige Tangentialkraft in Tangentialrichtung auf den Rotor ausgeübt wird,

Bestimmen voneinander unabhängiger Vorgaben für jede der von den Spulengruppen erzeugbaren Tangentialkräfte derart, dass aus den Tangentialkräften eine effektive Radialkraft zum Lagern des Rotors in Radialrichtung resultiert.

Das Verfahren kann entsprechend optionaler Merkmale der Steuereinheit, des Pumpensystems und/oder der Blutpumpe weitergebildet werden und/oder umgekehrt.

Eine implantierbare Blutpumpe der vorgeschlagenen Art umfasst einen Axialflussmotor mit einem Stator sowie einem magnetisch lagerbaren und zum Fördern von Blut um eine Rotationsachse rotierbaren Rotor, wobei der Stator mindestens drei, insbesondere genau drei, um die Rotationsachse herum angeordnete Spulengruppen umfasst, wobei jede der Spulengruppen eine Mehrzahl von benachbarten Statorspulen umfasst.

Es kann - wie bereits oben erwähnt - vorgesehen sein, dass die Blutpumpe eine passive Magnetlageranordnung zum passiven magnetischen Lagern des Rotors in mindestens einem ersten räumlichen Freiheitsgrad umfasst und der Rotor in mindestens den verbleibenden räumlichen Freiheitsgraden mittels der Statorspulen aktiv magnetisch lagerbar ist.

Alternativ oder zusätzlich kann vorgesehen sein, dass mindestens eine, vorzugsweise jede der Statorspulen, einen bezüglich einer zur Rotationsachse senkrechten Ebene abgewinkelten Wickelkopf umfasst. Auf diese Weise kann insbesondere eine Empfindlichkeit der Messung der Radialposition bei Verwendung des Rotors als Wirbelstromtarget (wie weiter oben beschrieben) verbessert werden.

Die nachfolgend beschriebenen Zeichnungen illustrieren Prinzipien und beispielhafte Ausführungsformen des Gegenstands der Anmeldung. Dabei zeigen, jeweils schematisch,

FIG. 1 ein Pumpensystem einschließlich einer Blutpumpe in Längsschnittansicht,

FIG. 2A und 2B Querschnittansichten eines Teils der Blutpumpe nach FIG. 1,

FIG. 3A und 3B beispielhafte Reglerstrukturen einer Steuereinheit,

FIG. 4A eine Querschnittsansicht eines Teils einer Blutpumpe nach einem weiteren Beispiel,

FIG. 4B eine Längsschnittsansicht eines Teils der Blutpumpe nach FIG. 4A,

FIG. 5, FIG. 6 und FIG. 7 Längsschnittsansichten von Teilen von Blutpumpen nach weiteren Beispielen,

FIG. 8 eine Schaltskizze für einen Teil des Pumpensystems nach FIG. 1.

Wiederkehrende und ähnliche Merkmale in den Zeichnungen sind mit identischen oder ähnlichen Bezugszeichen versehen. Diese können teilweise ausgelassen werden, wenn die entsprechenden Merkmale bereits in einer anderen Zeichnung gezeigt und im Zusammenhang mit dieser beschrieben werden oder wenn sie mit Bezug auf eine Zeichnung nicht erwähnt werden.

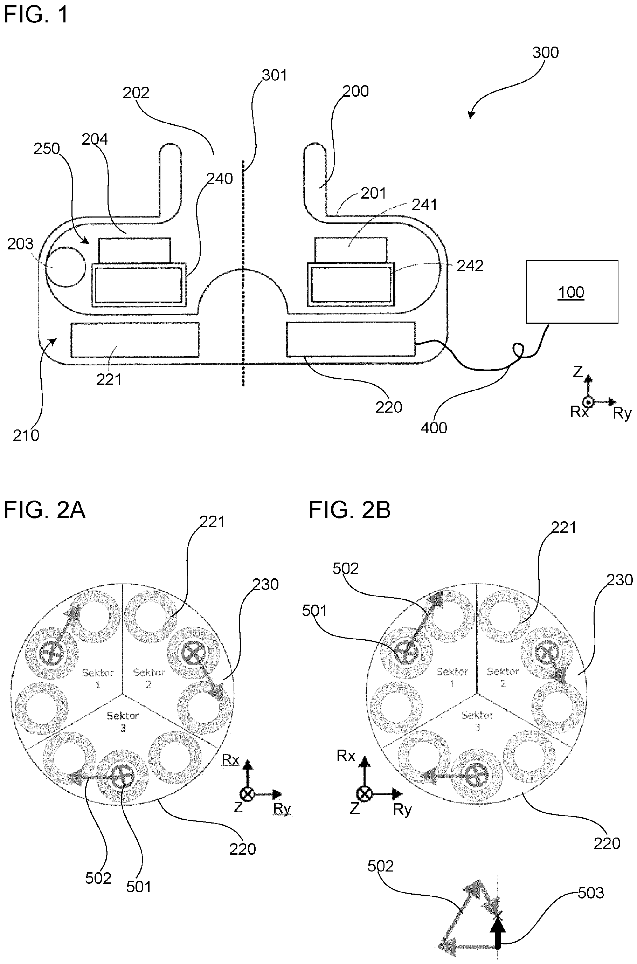

Das in FIG. 1 gezeigte Pumpensystem 300 umfasst eine Blutpumpe 200 und eine mit der Blutpumpe 200 mittels einer Driveline 400 verbundene Steuereinheit 100.

Die Blutpumpe 200 umfasst einen Axialflussmotor 210 mit einem Stator 220 sowie einem magnetisch lagerbaren und zum Fördern von Blut um eine Rotationsachse 301 rotierbaren Rotor 240. Der Stator 220 umfasst eine Mehrzahl von Statorspulen 221.

Der Stator 220 ist an einem Gehäuse 201 der Blutpumpe 200 angeordnet, das eine Kavität 204 bildet, in der der Rotor 240 aufgenommen ist. Das Gehäuse 201 umfasst einen Fluideinlass 202 und einen Fluidauslass 203, die mit jeweiligen Blutgefäßen und/oder einem Herzen fluidisch verbindbar sind.

Die Rotationsachse 301 ist die Hauptachse einer durch die Statorspulen 221 verursachbaren Rotation des Rotors, welche das Fluid antreibt. Wie im rechts unten eingezeichneten Koordinatensystem angedeutet, definiert die Rotationsachse 301 (in einer Soll-Position des Rotors) eine Axialrichtung Z sowie senkrecht dazu stehende Radialrichtungen Rx und Ry.

Der Rotor 240 umfasst eine Rotormagnetanordnung 242, die mit einem von den Statorspulen 221 erzeugten Magnetfeld wechselwirkt und so den Axialflussmotor 210 bildet.

Die Blutpumpe 200 ist im gezeigten Beispiel eine Zentrifugalpumpe, jedoch nicht darauf beschränkt.

Bei der Blutpumpe 200 handelt es sich um eine implantierbare Blutpumpe (implantierbares VAD), jedoch sind auch wenigstens teilweise extrakorporale Ausführungen denkbar. Die Steuereinheit 100 ist eine extrakorporale Steuereinheit. Die Steuereinheit kann jedoch auch ganz oder teilweise in die Blutpumpe integriert und/oder mit dieser implantierbar sein. Es kann auch vorgesehen sein, dass die Steuereinheit sowohl an der Blutpumpe angeordnete als auch extern/extrakorporal angeordnete Teile umfasst.

Wie in FIG. 2A und 2B erkennbar, umfasst der Stator drei koplanar um die Rotationsachse herum angeordnete Spulengruppen 230 (Sektormotoren bzw. Sektoren 1, 2 und 3), wobei jede der Spulengruppen 230 drei benachbarte und koplanar angeordnete Statorspulen 221 umfasst. Alternativ können die Spulengruppen beispielsweise so angeordnet sein, dass sie eine gemeinsame Ebene schneiden und gegenüber dieser abgewinkelt sind, und/oder so, dass sie annähernd die Form eines Kegelsegments bilden.

Die Statorspulen 221 jeder Spulengruppe 230 sind als Phasen des jeweiligen Sektormotors eingerichtet. Die Steuereinheit 100 kann zur entsprechenden Ansteuerung der Sektormotoren eingerichtet sein. Der Stator 220 kann eine andere Zahl von Spulengruppen, jede der Spulengruppen eine andere Zahl von Statorspulen umfassen als im Beispiel gezeigt.

Anhand von FIG. 2A bis 3B wird ein Verfahren zum Ansteuern einer Blutpumpe am Beispiel der Blutpumpe 200 illustriert.

Das Verfahren umfasst ein Bestimmen von Vorgaben für einen jeweiligen elektrischen Strom durch jede der Statorspulen 221 derart, dass durch den Stator 220 eine drei Axialkräfte 501 zum aktiven magnetischen Lagern des Rotors 240 in Axialrichtung Z auf den Rotor 240 ausgeübt wird und dass durch jede der Spulengruppen 230 eine jeweilige Tangentialkraft 502 in Tangentialrichtung auf den Rotor ausgeübt wird. Die gesamte Axialkraft auf den Rotor 240 entspricht dem Mittelwert der drei Axialkraftkomponenten 501.

Das Verfahren umfasst ferner ein Bestimmen voneinander unabhängiger Vorgaben für jede der von den Spulengruppen 230 erzeugbaren Tangentialkräfte 502 derart, dass aus den Tangentialkräften durch vektorielle Addition eine effektive Radialkraft 503 zum Lagern des Rotors 240 in Radialrichtung resultiert. In der in FIG. 2A gezeigten Konfiguration sind die drei Tangentialkräfte 502 dem Betrag nach gleich groß, so dass sich die Radialkomponenten zu Null addieren. Diese Situation kann etwa in einer Soll-Lage des Rotors 240 auftreten. In FIG. 2B haben die Tangentialkräfte 502 unterschiedliche Beträge. Die vektorielle Addition (unten in der Abbildung verdeutlicht) ergibt daher eine effektive Radialkraft 503, die auf den Rotor 240 wirkt. In beiden Fällen ergibt sich aus dem Mittelwert der Tangentialkräfte 502 ein Drehmoment bezüglich der Rotationsachse 301, um den Rotor 240 zum Fördern von Blut in Rotation zu versetzen bzw. die Rotation des Rotors zu regeln.

Zum Ausführen des beschriebenen Verfahrens ist die Steuereinheit 100 also dazu eingerichtet, Vorgaben für einen jeweiligen elektrischen Strom durch jede der Statorspulen 221 derart zu bestimmen, dass durch jede der Spulengruppen 230 eine jeweilige Tangentialkraft 502 in Tangentialrichtung auf den Rotor 240 ausgeübt wird. Die Steuereinheit 100 ermöglicht damit eine Radialregelung des Rotors 240 und dementsprechend ein aktiv magnetisches Lagern in Radialrichtung mit den Spulengruppen 230 des Axialflussmotors 210.

Die Steuereinheit 100 kann ferner für eine Axialregelung und Kippregelung des Rotors 240 eingerichtet sein. Hierzu ist es vorgesehen, dass jede Spulengruppe eine jeweilige Axialkraft 501 derart erzeugt, dass sich ein resultierendes Drehmoment bezüglich der zur Rotationsachse 301 senkrechten Achsen ergibt, das zum Regeln der Verkippung steuerbar ist. Aus den einzelnen Axialkräften 501 resultiert auch eine effektive Axialkraft zum aktiven magnetischen Lagern des Rotors 240 in Axialrichtung. Somit kann der Rotor 240 in allen Freiheitsgraden allein durch die Sektormotoren des Axialflussmotors 210 aktiv magnetisch gelagert werden.

FIG. 3A und 3B zeigen verschiedene mögliche Reglerkonfigurationen der Steuereinheit 100.

Gemäß der Reglerkonfiguration nach FIG. 3A ist die Steuereinheit 100 eingerichtet zum Regeln der Position und der Rotation des Rotors 240 mittels eines Reglers für eine effektive Axialposition des Rotors (Axialpositionsregler) bezüglich des Stators 220 insgesamt, zweier Regler für jeweilige Kippwinkel des Rotors 240 (Kippregler) bezüglich jeweiliger zur Rotationsachse 301 senkrechten Kippachsen, zweier Radialpositionsregler für die beiden Freiheitsgrade senkrecht zur Rotationsache 301 sowie einem Drehgeschwindigkeitsregler.

Die Steuereinheit 100 ist dazu eingerichtet, die Vorgaben für jede der von den Spulengruppen 230 erzeugbaren Tangentialkräfte 502 und Liftkräfte 501 mittels einer Transformationsmatrix aus Kraftvorgaben ($F\_x$, $F\_y$, $F\_z$) und Drehmomentvorgaben ($M\_x$, $M\_y$, $M\_z$) bezüglich kartesischer Koordinaten Rx, Ry, Rz (wie in den jeweiligen Koordinatensystemen in FIG. 1, FIG. 2A und FIG. 2B gezeigt) zu bestimmen. Mit den transformierten Vorgaben werden jeweilige Kommutatoren oder FOC-Blöcke beaufschlagt, die zur Ausgabe von Steuersignalen zum Ansteuern der Statorspulen 221 (insbesondere der genannten Vorgaben für einen jeweiligen elektrischen Strom durch jede der Statorspulen 221) eingerichtet sind. Die Vorgaben für Ströme können auch als Vorgaben für anliegende Spannungen vorliegen, aus denen sich resultierende Ströme ergeben. Die Phasenströme in den einzelnen Statorspulen können gemessen werden und mit einer Vektorregelung nachgestellt werden. Alternativ können die Ströme auch ohne Rückkopplung über die Messung, dann insbesondere mittels Sinuskommutierung, eingeprägt werden. Lediglich bei der der Antriebsmomenterzeugung entsteht eine große Gegeninduktion, welche den Strom hemmt. Dies kann jedoch bei bekannter Drehzahl in der Sinuskommutierung oder dem Drehzahlregler kompensiert werden.

Jeweilige Statorspulen entsprechen den Phasen Ui, Vi, Wi eines gegebenen Sektormotors (Spulengruppe) mit Index i (i läuft in diesem Fall von 1 bis 3). Wie erwähnt sind abweichende Anzahlen von Spulengruppen und/oder Statorspulen je Spulengruppe möglich.

Gemäß der Reglerkonfiguration nach FIG. 3B ist die Steuereinheit 100 eingerichtet zum Regeln einer Position des Rotors 240 mittels jeweiliger Regler für jeweilige Axialpositionen des Rotors (drei Axialpositionsregler) bezüglich jedes der Sektormotoren (also für jeweilige, voneinander unabhängige Liftkräfte), womit Kippwinkel des Rotors bezüglich zur Rotationsachse senkrechter Kippachsen festliegen, also nicht unabhängig geregelt werden. Hinsichtlich der Radialregelung und Drehgeschwindigkeitsregelung sowie der eigentlichen Ansteuerung mittels Kommutatoren/FOC-Blöcken entspricht die Konfiguration derjenigen nach FIG. 3A. Allerdings ist die Transformationsmatrix in diesem Fall kleiner, da nur die beiden Kräfte F_x und F_y sowie das Drehmoment M_z zu transformieren sind.

Alternativ zu einer Regelung der im Vorausgehenden beschriebenen Art kann die Erzeugung von Radialkräften bzw. Axialkräften auch dazu verwendet werden, um lediglich bekannte radiale Strömungskräfte und/oder Kippmomente zu kompensieren. Die Steuereinheit 100 kann dementsprechend eingerichtet sein zum Bestimmen der Vorgaben für jede der von den Spulengruppen erzeugbaren Tangentialkräfte derart, dass eine auf den Rotor wirkendende äußere Radialkraft und/oder ein auf den Rotor wirkendes äußeres Kippmoment ohne aktive Regelung kompensiert wird. Die äußere Radialkraft bzw. das äußere Kippmoment kann dabei insbesondere aus einer basierend auf einer Flussmessung und/oder Flussschätzung bestimmbaren Strömungskraft resultieren.

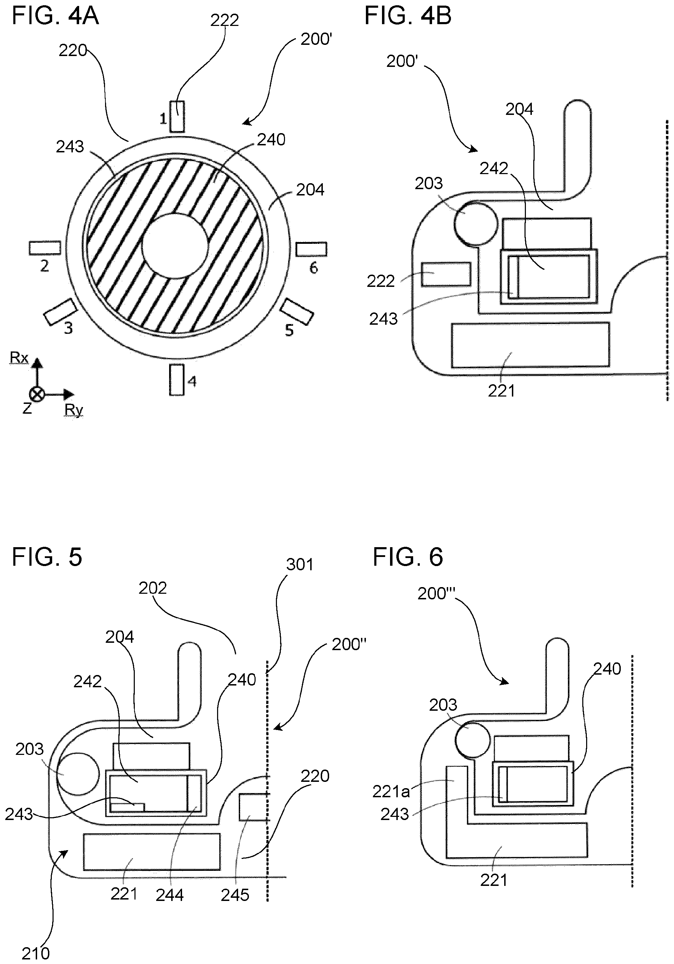

Zum Ermöglichen einer Regelung wie oben beschrieben ist ferner ein Bestimmen der Position des Rotors 240 vorgesehen. Beispiele für geeignete Anordnungen, die insbesondere das Bestimmen der Radialposition und/oder Verkippung ermöglichen, sind in FIG. 4A bis FIG. 6 dargestellt.

Der Stator 220 der in FIG. 4A und 4B gezeigten Blutpumpe 200' umfasst eine Anordnung von Wirbelstromsensoren 222, die Radial um den Rotor 240 herum im Gehäuse 201 angeordnet sind. Der Rotor 240 umfasst ein ringförmiges Wirbelstromtarget 242, das mit den Wirbelstromsensoren 222 wechselwirkt und so die Positionsbestimmung (x- und y-Position) ermöglicht.

Hierbei sind mit den Ziffern 1 bis 6 mögliche Positionen der Wirbelstromsensoren 222 gezeigt, von denen jedoch - je nach vorgesehenem Messverfahren - nur ein Teil mit Wirbelstromsensoren 222 belegt sein kann. Wichtig ist hierbei jeweils, dass x- und y-Position des Rotors 240 bestimmt werden kann. Hierfür sind Sensoranordnungen mit beispielsweise zwei um 90° verschobenen Sensoren oder drei oder vier auf dem Umfang verteilten Sensoren denkbar.

Für eine einkanalige Messung können Wirbelstromsensoren 222 an den Positionen 1 und 2 angeordnet sein.

Für eine differentielle Messung können Wirbelstromsensoren 222 an den Positionen 1 und 4 für die y-Position sowie 2 und 6 für die x-Position angeordnet sein. Eine differentielle Messung zeichnet sich gegenüber einer einkanaligen Messung durch bessere Unempfindlichkeit gegenüber der Rotorgröße oder Störeinflüssen aus.

Für eine dreiphasige Messung können Wirbelstromsensoren 222 an den Positionen 1, 3 und 5 angeordnet sein. Eine dreiphasige Messung hat dieselben Vorteile wie eine differentielle Messung, benötigt zusätzlich aber weniger Sensoren und ist daher besonders bevorzugt.

Alternativ zu einem dedizierten Wirbelstromsensor (wie in FIG. 4A und 4B gezeigt) kann ein Wirbelstromsensor durch eine oder mehrere der Statorspulen 221 gebildet sein, wie in FIG. 5 und 6 gezeigt. Somit wird eine Positionsmessung ohne zusätzliche Sensorik ermöglicht. Die in FIG. 5 gezeigte Blutpumpe 200" umfasst ebenfalls ein am Rotor 240 angeordnetes ringförmiges Wirbelstromtarget 243, das in diesem Fall jedoch nicht radial außen (wie in FIG. 4A/B), sondern zu dem Statorspulen 221 hin am Rotor 240 angeordnet ist.

Zusätzlich oder alternativ zu der Positionsmessung mit Wirbelstrommessung kann die Steuereinheit 100 eingerichtet sein zum Bestimmen der Rotorposition basierend auf einer erfassten elektromotorischen Gegenkraft (Back-EMF), die durch die Rotation des Rotors in den Statorspulen induziert wird. Von jedem Sektormotor kann dabei eine dreiphasige Back-EMF gemessen werden, die ohne Informationsverlust in einen zweidimensionalen Vektor transformiert werden, aus dem die Positionsinformation bestimmbar ist.

Besonders vorteilhaft ist eine Kombination beider Messverfahren (Back-EMF-Messung und Wirbelstrommessung mit Statorspulen), die zudem auf die Verwendung des Wirbelstromtargets 243 mit einem wirksamen Radius, der größer ist (oder jedenfalls unterschiedlich) als ein wirksamer Radius der Rotormagnetanordnung 242, gestützt ist. Durch diese Anordnung kann erreicht werden, dass beide Messverfahren unterschiedlich empfindlich auf x- und y-Abweichungen reagieren, wodurch die Positionsbestimmung verbessert wird, da ansonsten eine Verkippung nur ungenau von einer radialen Verschiebung unterschieden werden kann.

FIG. 5 zeigt zusätzlich eine passive Magnetlageranordnung zum passiven magnetischen Lagern des Rotors in der Radialrichtung, umfassend eine erste Magnetlagerkomponente 244 und eine zweite Magnetlagerkomponente 245. Die erste Magnetlagerkomponente 244 ist dabei radial innen am Rotor 240 angeordnet, die zweite Magnetlagerkomponente 245 am Stator 220 derart, dass sie in einen axial angeordneten Hohlraum des Rotors 240 hineinragt und von der Rotormagnetanordnung 242 wenigstens teilweise umgeben ist. Obwohl die passive Magnetlageranordnung im Zusammenhang mit der Blutpumpe 200" illustriert ist, kann sie bei dieser auch weggelassen und/oder bei einer anderen Gestaltung der Blutpumpe, etwa bei den Blutpumpen 200, 200' und/oder 200''', vorgesehen sein. Die passiv gelagerten Freiheitsgrade können zusätzlich durch eine aktive Regelung im Bewegungsraum begrenzt, oder durch eine aktive Dämpfung am Aufschwingen gehindert, werden. Der Einbau des passiven Magnetlagers ermöglicht zudem eine Nullkraftregelung (auch Nullleistungsregelung, in englischer Fachliteratur Zero Power Control) in den passiv magnetisch instabilen Freiheitsgraden. Durch die Nullkraftregelung können statische Kräfte, wie beispielsweise die Gewichtskraft des Rotors, allein von den passiven Magnetlagerkomponenten aufgenommen werden. Dies reduziert die Kräfte, die durch Strom- und damit Leistungsverluste in den Statorspulen aufgebracht werden müssen.

Bei der in FIG. 6 gezeigten Blutpumpe 200''' umfasst jede der Statorspulen 221 einen in Axialrichtung zum Rotor 240 hin abgewinkelten Wickelkopf 221a. Auf diese Weise kann die Radialempfindlichkeit der Wirbelstrommessung mittels Statorspulen erhöht werden. Eine solche Anordnung kann sowohl in Verbindung mit einem dedizierten Wirbelstromsensor (wie bei Blutpumpe 200') als auch mit der Back-EMF-Messung (wie bei Blutpumpe 200") vorgesehen sein. Das Wirbelstromtarget 243 kann in diesem Fall insbesondere radial außen am Rotor 240 angeordnet sein. Die separaten Wirbelstromsensoren können optional zu den Statorspulen parallelgeschaltet werden, um diese über dieselben Anschlussleitungen auszulesen. Die Antriebssignale der Sektormotoren und der Back-EMF kann im Frequenzbereich vom Wirbelstromsignal getrennt werden.

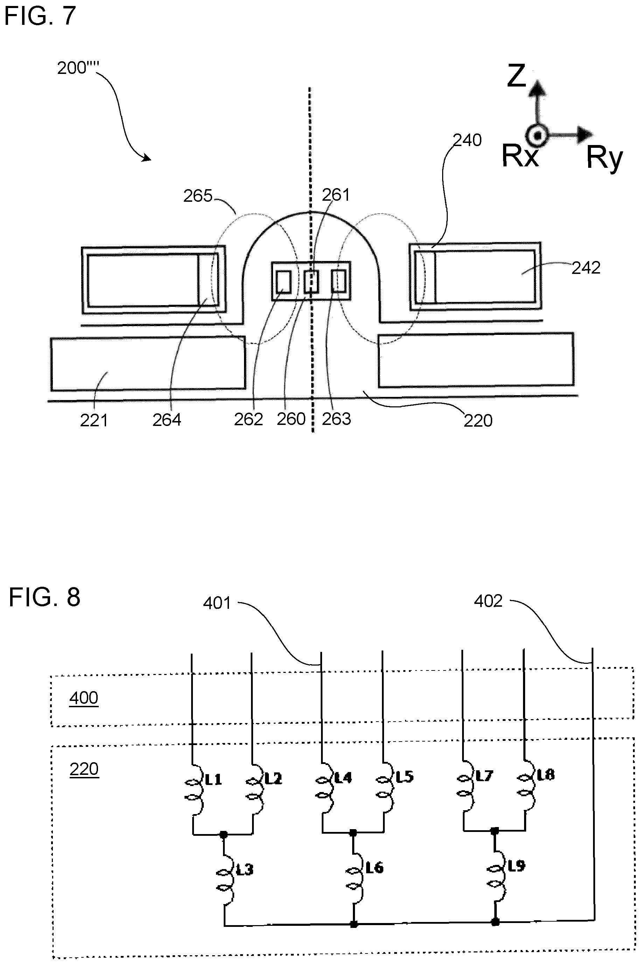

Bei der in FIG. 7 gezeigten Blutpumpe 200'''' erfolgt die Bestimmung der Rotorposition basierend auf einer magnetischen Feldmessung mittels einer Magnetfeldsensoranordnung 260. Hierbei ist eine Targetmagnetanordnung 264 radial innen am Rotor 240 angeordnet. Am Stator 220 ist die Magnetfeldsensoranordnung 260 derart angeordnet, dass sie in einem axial angeordneten Hohlraum des Rotors 240 angeordnet und von der Targetmagnetanordnung 264 wenigstens teilweise umgeben ist. Ein Magnetfeld 265 der Targetmagnetanordnung 264 ist torusförmig und verläuft auf der Rotationsachse 301 in in Z-Richtung (wenn der Rotor 240 in seiner nicht verkippten Sollposition ist). Bei einer Verkippung des Rotors 240 entstehen an einem ersten Magnetfeldsensor 2361 von Null verschiedene Feldkomponenten in x- und y-Richtung, aus denen sich die Verkippung bestimmen lässt. Bei einer radialen Verschiebung des Rotors 240 entsteht am zweiten Magnetfeldsensor 262 eine andere Feldkomponente in z-Richtung als am dritten Magnetfeldsensor 263. Ist, wie bei der Blutpumpe 200" in FIG. 5 eine passive Magnetlageranordnung vorgesehen, so kann die Targetmagnetanordnung 264 mit der ersten Magnetlagerkomponente 244 übereinstimmen.

Eine Vermessung des magnetischen Feldes ist alternativ auch ohne Targetmagnetanordnung 264 möglich. Hierbei wird das Feld der Rotormagnetanordnung 242 selbst mittels einer Magnetfeldsensoranordnung vermessen. In diesem Fall können allerdings Inhomogenitäten des Magnetfelds die Messung beeinträchtigen. Eine Korrektur solcher Inhomogenitäten ist allerdings möglich, wie beispielsweise im Dokument EP 4 249 039 A1 beschrieben.

FIG. 8 zeigt ein mögliches Anschlussschema für die Statorspulen im Stator 220, die hier mit ihren Induktivitäten L1-L3 (erste Spulengruppe), L4-L6 (zweite Spulengruppe) und L7-L9 (dritte Spulengruppe) beschriftet sind. Die Driveline 400, mittels der die Steuereinheit 100 mit der Blutpumpe 200 verbindbar oder verbunden ist, umfasst sechs separate Anschlussleitungen 401 sowie eine gemeinsame Anschlussleitung 402. Die Steuereinheit 100 ist mit jeweils zwei Statorspulen (hier L1/L2, L4/L5, L7/L8) jeder Spulengruppe mittels der separaten Anschlussleitungen 401 elektrisch verbindbar oder verbunden. Mit den jeweils verbleibenden Statorspulen (hier L3, L6, L9) jeder Spulengruppe ist die Steuereinheit 100 mittels der gemeinsamen Anschlussleitung 402 elektrisch verbindbar oder verbunden. Die Driveline 400 kann, je nach Anwendung, neben den genannten Anschlussleitungen 401, 402 optional weitere Leitungen umfassen.

Liste der Bezugszeichen

| | |
|---|---|
| 100 | Steuereinheit, |
| 200, 200', 200", 200''', 200'''' | Blutpumpe, |
| 201 | Gehäuse, |
| 202 | Fluideinlass, |
| 203 | Fluidauslass, |
| 204 | Kavität, |
| 210 | Axialflussmotor, |
| 220 | Stator, |
| 221 | Statorspule, |

| | |
|---|---|
| 221a | abgewinkelter Wickelkopf, |
| 222 | Wirbelstromsensor, |
| 230 | Spulengruppe, |
| 240 | Rotor, |
| 241 | Beschaufelung, |
| 242 | Rotormagnetanordnung, |
| 243 | Wirbelstromtarget, |
| 244 | erste Magnetlagerkomponente, |
| 245 | zweite Magnetlagerkomponente, |
| 250 | Impeller, |
| 260 | Magnetfeldsensoranordnung, |
| 261 | erster Magnetfeldsensor, |
| 262 | zweiter Magnetfeldsensor, |
| 263 | dritter Magnetfeldsensor, |
| 264 | Targetmagnetanordnung, |
| 265 | Targetmagnetfeld, |
| 300 | Pumpensystem, |
| 301 | Rotationsachse, |
| 400 | Driveline, |
| 401 | separate Anschlussleitung, |
| 402 | gemeinsame Anschlussleitung, |
| 501 | Axialkraft, |
| 502 | Tangentialkraft, |
| 503 | effektive Radialkraft. |

**Patentansprüche**

1. Steuereinheit (100) für eine, insbesondere implantierbare, Blutpumpe (200),

   wobei die Blutpumpe (200) einen Axialflussmotor (210) mit einem Stator (220) sowie einem magnetisch lagerbaren und zum Fördern von Blut um eine Rotationsachse rotierbaren Rotor (240) umfasst, wobei der Stator (220) mindestens drei, insbesondere genau drei, um die Rotationsachse herum angeordnete Spulengruppen (230) umfasst, wobei jede der Spulengruppen (230) eine Mehrzahl von benachbarten Statorspulen (221) umfasst, wobei die Steuereinheit (100) dazu eingerichtet ist, Vorgaben für einen jeweiligen elektrischen Strom durch jede der Statorspulen (221) derart zu bestimmen, dass
   durch den Stator (220) eine Axialkraft (501) zum aktiven magnetischen Lagern des Rotors (240) in Axialrichtung auf den Rotor (240) ausgeübt wird,
   durch jede der Spulengruppen (230) eine jeweilige Tangentialkraft (502) in Tangentialrichtung auf den Rotor (240) ausgeübt wird,
   wobei die Steuereinheit (100) zum Bestimmen voneinander unabhängiger Vorgaben für jede der von den Spulengruppen (230) erzeugbaren Tangentialkräfte derart eingerichtet ist, dass aus den Tangentialkräften eine effektive Radialkraft (503) zum Lagern des Rotors (240) in Radialrichtung resultiert.

2. Steuereinheit (100) nach Anspruch 1, wobei jede der Spulengruppen (230) mindestens drei, insbesondere genau drei, benachbarte Statorspulen (221) umfasst.

3. Steuereinheit (100) nach Anspruch 2, wobei die Steuereinheit (100) mit jeweils zwei Statorspulen (221) jeder Spulengruppe (230) mittels separater Anschlussleitungen (401) elektrisch verbindbar oder verbunden und mit den jeweils verbleibenden Statorspulen (221) jeder Spulengruppe (230) mittels einer gemeinsamen Anschlussleitung (402) elektrisch verbindbar oder verbunden ist.

4. Steuereinheit (100) nach einem der vorhergehenden Ansprüche, eingerichtet zum Vorgeben des jeweiligen elektrischen Stroms durch jede der Statorspulen derart, dass der Rotor (240) in drei rotatorischen und drei translatorischen Freiheitsgraden aktiv magnetisch gelagert ist.

5. Steuereinheit (100) nach einem der vorhergehenden Ansprüche, wobei die Blutpumpe (200) eine passive Magnetlageranordnung (244, 245) zum passiven magnetischen Lagern des Rotors (240) in mindestens einem ersten räumlichen Freiheitsgrad umfasst und die Steuereinheit (100) eingerichtet ist zum Vorgeben des jeweiligen elektrischen Stroms durch jede der Statorspulen derart, dass der Rotor (240) in den verbleibenden räumlichen Freiheitsgraden aktiv magnetisch gelagert ist.

6. Steuereinheit (100) nach einem der vorhergehenden Ansprüche, eingerichtet zum Regeln einer Position des Rotors (240) mittels eines Reglers für eine effektive Axialposition des Rotors (240) bezüglich des Stators (220) insgesamt sowie mindestens eines Reglers für einen Kippwinkel des Rotors (240) bezüglich einer zur Rotationsachse senkrechten Kippachse.

7. Steuereinheit (100) nach einem der Ansprüche 5 und 6, dazu eingerichtet, die Vorgaben für jede der von den Spulengruppen (230) erzeugbaren Tangentialkräfte mittels einer Transformationsmatrix aus Kraft- und/oder Drehmomentvorgaben bezüglich kartesischer Koordinaten zu bestimmen.

8. Steuereinheit (100) nach einem der vorhergehenden Ansprüche, eingerichtet zum Regeln einer Position, insbesondere einer Translation und/oder Rotation und/oder Verkippung, des Rotors (240) mittels einer Null-Leistungs-Regelung.

9. Steuereinheit (100) nach einem der vorhergehenden Ansprüche, eingerichtet zum Bestimmen der Vorgaben für jede der von den Spulengruppen (230) erzeugbaren Tangentialkräfte derart, dass eine auf den Rotor (240) wirkende äußere Radialkraft und/oder ein auf den Rotor (240) wirkendes äußeres Kippmoment ohne aktive Regelung kompensiert wird, wobei die äußere Radialkraft bzw. das äußere Kippmoment insbesondere aus einer basierend auf einer Flussmessung und/oder Flussschätzung bestimmbaren Strömungskraft resultiert.

10. Steuereinheit (100) nach einem der vorhergehenden Ansprüche, eingerichtet zum Bestimmen einer Position, insbesondere Radialposition und/oder Verkippung, des Rotors (240) basierend auf einer erfassten elektromotorischen Gegenkraft (Back-EMF) und/oder basierend auf einer magnetischen Feldmessung und zum Bestimmen der Vorgaben für jede der von den Spulengruppen (230) erzeugbaren Tangentialkräfte basierend auf der bestimmten Position.

11. Steuereinheit (100) nach einem der vorhergehenden Ansprüche, wobei der Stator (220) einen zum Bestimmen einer Position, insbesondere Radialposition und/oder Verkippung, des Rotors (240) eingerichteten Wirbelstromsensor (222), insbesondere einen dreiphasigen Wirbelstromsensor (222) und/oder einen aus einer oder mehrerer der Statorspulen (221) gebildeten Wirbelstromsensor (222), umfasst, wobei der Rotor (240) ein Wirbelstromtarget (243) bildet oder umfasst, das vorzugsweise einen wirksamen Radius aufweist, der sich von einem wirksamen Radius einer zum Wechselwirken mit den Statorspulen (221) eingerichteten Rotormagnetbaugruppe unterscheidet, insbesondere größer ist, und wobei die Steuereinheit (100) zum Bestimmen der Vorgaben für jede der von den Spulengruppen (230) erzeugbaren Tangentialkräfte basierend auf der bestimmten Position eingerichtet ist.

12. Pumpensystem, umfassend

eine, insbesondere implantierbare, Blutpumpe (200), die einen Axialflussmotor (210) mit einem Stator (220) sowie einem magnetisch lagerbaren und zum Fördern von Blut um eine Rotationsachse rotierbaren Rotor (240) umfasst, wobei der Stator (220) mindestens drei, insbesondere genau drei, um die Rotationsachse herum angeordnete Spulengruppen (230) umfasst, wobei jede der Spulengruppen (230) eine Mehrzahl von benachbarten Statorspulen (221) umfasst, und
eine Steuereinheit (100) nach einem der vorhergehenden Ansprüche.

13. Verfahren zum Ansteuern einer, insbesondere implantierbaren, Blutpumpe (200), die einen Axialflussmotor (210) mit einem Stator (220) sowie einem magnetisch lagerbaren und zum Fördern von Blut um eine Rotationsachse rotierbaren Rotor (240) umfasst, wobei der Stator (220) mindestens drei, insbesondere genau drei, koplanar um die Rotationsachse herum angeordnete Spulengruppen (230) umfasst, wobei jede der Spulengruppen (230) eine Mehrzahl von benachbarten und koplanar angeordneten Statorspulen (221) umfasst, wobei das Verfahren umfasst:

Bestimmen von Vorgaben für einen jeweiligen elektrischen Strom durch jede der Statorspulen (221) derart, dass durch den Stator (220) eine Axialkraft (501) zum aktiven magnetischen Lagern des Rotors (240) in Axialrichtung auf den Rotor (240) ausgeübt wird,
durch jede der Spulengruppen (230) eine jeweilige Tangentialkraft (502) in Tangentialrichtung auf den Rotor (240) ausgeübt wird,
Bestimmen voneinander unabhängiger Vorgaben für jede der von den Spulengruppen (230) erzeugbaren Tangentialkräfte derart, dass aus den Tangentialkräften eine effektive Radialkraft (503) zum Lagern des Rotors (240) in Radialrichtung resultiert.

14. Implantierbare Blutpumpe (200), umfassend einen Axialflussmotor (210) mit einem Stator (220) sowie einem magnetisch lagerbaren und zum Fördern von Blut um eine Rotationsachse rotierbaren Rotor (240), wobei der Stator (220) mindestens drei, insbesondere genau drei, koplanar um die Rotationsachse herum angeordnete Spulengruppen (230) umfasst, wobei jede der Spulengruppen (230) eine Mehrzahl von benachbarten und koplanar angeordneten Statorspulen (221) umfasst,
wobei die Blutpumpe (200) eine passive Magnetlageranordnung zum passiven magnetischen Lagern des Rotors (240) in mindestens einem ersten räumlichen Freiheitsgrad umfasst und der Rotor (240) in mindestens den verbleibenden räumlichen Freiheitsgraden mittels der Statorspulen (221) aktiv magnetisch lagerbar ist.

15. Implantierbare Blutpumpe (200), umfassend einen Axialflussmotor (210) mit einem Stator (220) sowie einem magnetisch lagerbaren und zum Fördern von Blut um eine Rotationsachse rotierbaren Rotor (240), wobei der Stator (220) mindestens drei, insbesondere genau drei, koplanar um die Rotationsachse herum angeordnete Spulengruppen (230) umfasst, wobei jede der Spulengruppen (230) eine Mehrzahl von benachbarten und koplanar angeordneten Statorspulen (221) umfasst,
wobei mindestens eine, vorzugsweise jede der Statorspulen (221) einen bezüglich einer zur Rotationsachse senkrechten Ebene abgewinkelten Wickelkopf (221a) umfasst.

# FIG. 1

# FIG. 2A

# FIG. 2B

FIG. 3A

Radialpositionsregler

Radialpositionsregler

Axialpositionsregler

Kippregler

Kippregler

Drehgeschwin-digkeitsregler

$\begin{pmatrix} F\_x \\ F\_y \\ F\_z \\ M\_x \\ M\_y \\ M\_z \end{pmatrix}$

Matrix-multiplikation mit Tranformations-matrix

$\begin{pmatrix} Liftkraft\ 1 \\ Tangentialkraft\ 1 \\ Liftkraft\ 2 \\ Tangentialkraft\ 2 \\ Liftkraft\ 3 \\ Tangentialkraft\ 3 \end{pmatrix}$

Kommutator oder FOC — Phase U1 / Phase V1 / Phase W1 — Sektor-Motor 1

Kommutator oder FOC — Phase U2 / Phase V2 / Phase W2 — Sektor-Motor 2

Kommutator oder FOC — Phase U3 / Phase V3 / Phase W3 — Sektor-Motor 3

FIG. 3B

Radialpositionsregler

Radialpositionsregler

Drehgeschwin-digkeitsregler

$\begin{pmatrix} F\_x \\ F\_y \\ M\_z \end{pmatrix}$

Matrix-multiplikation mit Tranformations-matrix

$\begin{pmatrix} Tangentialkraft\ 1 \\ Tangentialkraft\ 2 \\ Tangentialkraft\ 3 \end{pmatrix}$

Axialpositionsregler

Axialpositionsregler

Axialpositionsregler

Kommutator oder FOC — Phase U1 / Phase V1 / Phase W1 — Sektor-Motor 1

Kommutator oder FOC — Phase U2 / Phase V2 / Phase W2 — Sektor-Motor 2

Kommutator oder FOC — Phase U3 / Phase V3 / Phase W3 — Sektor-Motor 3

14

FIG. 4A

FIG. 4B

FIG. 5

FIG. 6

FIG. 7

FIG. 8

Nummer der Anmeldung

EP 24 18 8330

Europäisches
Patentamt

European
Patent Office

Office européen
des brevets

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 063 753 A1 (SULZER ELECTRONICS AG [CH]; LUST ANTRIEBSTECHNIK GMBH [DE]) 27. Dezember 2000 (2000-12-27) | 1-9,11, 12 | INV. A61M60/122 A61M60/216 |
| Y | * Absatz [0008] * * Absatz [0025] * * Absatz [0036] * * Absatz [0049] * * Absatz [0073] - Absatz [0074] * * Absatz [0078] * * Absatz [0039] - Absatz [0040]; Abbildung 2 * ----- | 10 | A61M60/232 A61M60/422 A61M60/508 A61M60/822 F16C32/04 H02K7/09 |
| A | SUN CHAO ET AL: "An Improved Mathematical Model for Speed Sensorless Control of Fixed Pole Bearingless Induction Motor", IEEE TRANSACTIONS ON INDUSTRIAL ELECTRONICS, IEEE SERVICE CENTER, PISCATAWAY, NJ, USA, Bd. 71, Nr. 2, 16. März 2023 (2023-03-16), Seiten 1286-1295, XP011947182, ISSN: 0278-0046, DOI: 10.1109/TIE.2023.3247754 [gefunden am 2023-03-16] * das ganze Dokument * ----- | 7 | |
| Y | EP 4 249 040 A1 (BERLIN HEART GMBH [DE]) 27. September 2023 (2023-09-27) * Absatz [0053] - Absatz [0054] * ----- | 10 | RECHERCHIERTE SACHGEBIETE (IPC) A61M F16C H02K |

Der vorliegende Recherchenbericht wurde für alle Patentansprüche erstellt

| Recherchenort | Abschlußdatum der Recherche | Prüfer |
|---|---|---|
| Den Haag | 19. Dezember 2024 | Weiss-Schaber, C |

KATEGORIE DER GENANNTEN DOKUMENTE

X : von besonderer Bedeutung allein betrachtet
Y : von besonderer Bedeutung in Verbindung mit einer
      anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
      nach dem Anmeldedatum veröffentlicht worden ist
D : in der Anmeldung angeführtes Dokument
L : aus anderen Gründen angeführtes Dokument
...........................................................................................
& : Mitglied der gleichen Patentfamilie, übereinstimmendes
      Dokument

EPO FORM 1503 03.82 (P04C03)

Europäisches
Patentamt

European
Patent Office

Office européen
des brevets

---

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

---

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

**Siehe Ergänzungsblatt B**

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

**1-12**

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches
Patentamt

European
Patent Office

Office européen
des brevets

**MANGELNDE EINHEITLICHKEIT
DER ERFINDUNG
ERGÄNZUNGSBLATT B**

**Nummer der Anmeldung**

**EP 24 18 8330**

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-12

   Steuereinheit für eine Blutpumpe (100) zum Bestimmen voneinander unabhängiger Vorgaben für jede der von mindestens drei Spulengruppen (230) erzeugbaren Tangentialkräfte.
   ---

2. Ansprüche: 13-15

   Implantierbare Blutpumpe (200), umfassend einen Axialflussmotor (210) mit einem Stator (220), der mindestens drei koplanar um die Rotationsachse herum angeordnete Spulengruppen (230) umfasst, wobei jede der Spulengruppen (230) eine Mehrzahl von benachbarten und koplanar angeordneten Statorspulen (221) umfasst
   ---

**ANHANG ZUM EUROPÄISCHEN RECHERCHENBERICHT
ÜBER DIE EUROPÄISCHE PATENTANMELDUNG NR.**

EP 24 18 8330

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-12-2024

| Im Recherchenbericht angeführtes Patentdokument | Datum der Veröffentlichung | Mitglied(er) der Patentfamilie | Datum der Veröffentlichung |
|---|---|---|---|
| EP 1063753 A1 | 27-12-2000 | EP 1063753 A1 | 27-12-2000 |
| | | JP 4484320 B2 | 16-06-2010 |
| | | JP 2001016887 A | 19-01-2001 |
| | | US 6351048 B1 | 26-02-2002 |
| EP 4249040 A1 | 27-09-2023 | CN 118891081 A | 01-11-2024 |
| | | DE 112023001469 A5 | 02-01-2025 |
| | | EP 4249040 A1 | 27-09-2023 |
| | | WO 2023180325 A1 | 28-09-2023 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3827852 A1
- EP 24153240 A
- EP 4249039 A1